# EUROPEAN PATENT SPECIFICATION

(11) **EP 3 236 871 B1**
(45) Date of publication and mention of the grant of the patent: **14.11.2018**
(21) Application number: 15825720.4
(22) Date of filing: 21.12.2015
(51) Int. Cl.: A61B 18/14

(54) **ELECTROSURGICAL FORMATION OF PSEUDOPOLYPS**
ELEKTROCHIRURGISCHE BILDUNG VON PSEUDOPOLYPEN
FORMATION ÉLECTROCHIRURGICALE DE PSEUDOPOLYPES

(30) Priority: 23.12.2014 US 201462095998 P
(43) Date of publication of application: 01.11.2017
(73) Proprietor: Cook Medical Technologies LLC, Bloomington, IN 47402 (US)
(72) Inventor: MCLAWHORN, Tyler Evans, Winston-Salem, NC 27106 (US)
(74) Representative: Garratt, Peter Douglas
(86) International application number: PCT/US2015/067058
(87) International publication number: WO 2016/106200

(56) References cited:
- US-A1- 2004 158 124
- US-A1- 2004 158 127
- US-A1- 2014 276 774

## Description

### TECHNICAL FIELD

The present invention relates generally to medical devices and more particularly to electrosurgical medical systems of withdrawing a target tissue portion into a distal chamber and applying an electrical current to the target tissue portion without detaching the target tissue portion from underlying tissue.

### BACKGROUND

To perform piecemeal endoscopic mucosal resection (EMR), a multi-band mucosectomy device may be delivered to a treatment site where it suctions target tissue into a cap mounted onto a distal end of an endoscope, which forms the target tissue into a pseudopolyp or raised legion. With the target tissue suctioned into the cap and in a raised position, a band may be deployed over a base of the target tissue. The band may maintain the target tissue in the raised position as a pseudopolyp when the suction is removed. A snare may then be used to resect the target tissue in the raised position.

A mucosectomy device may include a limited amount of preloaded bands. If a physician operating the mucosectomy device wish to deploy more bands than are preloaded, the mucosectomy device has to be withdrawn from the patient in order to deploy a device with more bands. The document US2004/0158124 discloses an electrosurgical system according to the preamble of claim 1.

### BRIEF SUMMARY

By way of introduction, the below summary relates to electrosurgical medical systems and methods for withdrawing a target tissue portion into a distal chamber and applying an electrical current to the target tissue portion while the target tissue portion is being withdrawn into the chamber without detaching the target tissue portion from underlying tissue. An electrosurgical method may include positioning a distal end of an elongate tubular member adjacent a target tissue portion of underlying tissue at a treatment site; and applying a proximal bias to the target tissue portion to withdraw the target tissue portion into a distal chamber of the elongate tubular member without detaching the target tissue portion from the underlying tissue. The target tissue portion is in a raised position when withdrawn into the distal chamber. In addition, the method may include, while applying the proximal bias, applying, with an electrode assembly, an electrical current to the target tissue portion in the raised position to coagulate the target tissue portion without detaching the target tissue portion from the underlying tissue.

Applying the proximal bias may include applying, with a suction source, a suction in a suction lumen of the elongate tubular member. The suction lumen is in fluid communication with the distal chamber.

Applying the electrical current to coagulate the target tissue portion creates a scarring at a base of the target tissue portion. The method may further include ceasing application of the proximal bias and the electrical current after creating the scarring at the base, where the scarring at the base maintains the target tissue portion in the raised position.

Applying the electrical current includes applying, with the electrode assembly, the electrical current to the base such that the base coagulates in response to the electrical current. The scarring at the base occurs as a result of the coagulation.

The electrode assembly may include at least one ring-shaped electrode, where applying the electrical current to the base may include circumferentially applying, with the at least one ring-shaped electrode, the electrical current to the base.

The method may further include detaching, with a detachment device, the target tissue portion from the underlying tissue. In some of those embodiments, the detachment device may include an electrosurgical snare.

The method may further include: deploying a distal loop portion of an electrosurgical snare to within the distal chamber before the target tissue portion is withdrawn into the distal chamber such that when the target tissue portion is withdrawn into the distal chamber, the target tissue portion passes through the distal loop portion. The method may further include, after the scarring at the base is created, detaching the target tissue portion from the underlying tissue by applying, with the distal loop portion, a second electrical current to the target tissue portion while withdrawing the distal loop portion out of the distal chamber.

In some examples, the electrode assembly has either a monopolar or a bipolar configuration.

In some examples, prior to applying the electrical current, the target tissue portion is bleeding, and applying the electrical current to the target tissue portion in the raised position coagulates the target tissue portion in order to stop the bleeding.

In some examples, the underlying tissue may include a gastrointestinal wall, and the target tissue portion in the distal chamber includes at most a mucosa layer and a submucosa layer of the gastrointestinal wall.

According to the invention, an electrosurgical system includes an elongate tubular member extending from a proximal portion to a distal portion. The tubular member includes a body longitudinally extending from the proximal portion to the distal portion; a distal chamber disposed within the body at the distal portion; and a snare lumen longitudinally extending in the body and in fluid communication with the distal chamber. In addition, the electrosurgical system includes a deployable electrosurgical snare comprising a distal loop portion that is movable between an undeployed position and a deployed position. In the undeployed position, the distal loop portion is at least partially disposed in the snare lumen extending in the body, and in the deployed position, the distal loop portion is disposed within the distal chamber circumferentially adjacent an inner surface of the body defining the distal chamber. The system also includes a return electrode in the distal chamber that is at least partially circumferentially disposed on the inner surface. When the distal loop portion is deployed in the deployed position, the distal loop portion and the return electrode are disposed at a distal portion of distal chamber for contact with a base of a target tissue portion of underlying tissue that is withdrawn into the distal chamber.

In some embodiments, the elongate tubular further includes a suction lumen longitudinally extending in the body. The distal chamber is in fluid communication with and disposed distal the suction lumen.

The some embodiments, the distal loop portion, in the deployed position, is disposed distal the return electrode.

In some embodiments, the elongate tubular member further includes a lip that decreases a diameter of the distal chamber at a distal opening of the distal chamber to prevent the distal loop portion from slipping outside of the distal chamber.

In some embodiments, the return electrode includes a ring-shaped structure.

In some embodiments, when the distal loop portion is in the deployed position, the distal loop portion and the return electrode are oriented in substantially parallel planes.

Other embodiments are possible, and each of the embodiments can be used alone or together in combination. Accordingly, various embodiments are described below with reference to the attached drawings.

### BRIEF DESCRIPTION OF THE DRAWINGS

FIG. 1 is a partial cross-sectional side view of an example medical system that includes an elongate tubular electrosurgical device.
FIG. 2A is a partial cross-sectional side view of the example medical system of Fig. 1, showing a distal portion of the electrosurgical device being delivered to a treatment site.
FIG. 2B is a partial cross-sectional side view of the example medical system of Fig. 1, showing a target tissue portion being withdrawn into a distal chamber of the electrosurgical device.
FIG. 2C is a partial cross-sectional side view of the example medical system of Fig. 1, showing a bipolar electrode assembly applying current to a base of the target tissue portion.
FIG. 2D is a partial cross-sectional side view of the example medical system of Fig. 1, showing a proximal bias and electrical current being removed from the base after the base has sufficiently scarred.
FIG. 2E is a partial cross-sectional side view of the example medical system of Fig. 1, showing an electrosurgical snare being delivered to the treatment site.
FIG. 2F is a partial cross-sectional side view of the example medical system of Fig. 1, showing the electrosurgical snare being positioned around the base.
FIG. 2G is a partial cross-sectional side view of the example medical system of Fig. 1, showing the electrosurgical snare detaching the target tissue portion from the underlying tissue.
FIG. 3 is a partial cross-sectional side view of another example medical system that includes an electrosurgical device having a monopolar electrode assembly.
FIG. 4A is a partial cross-sectional side view of another example medical system that includes an electrosurgical device having a deployable electrosurgical snare, showing the snare in the undeployed position.
FIG. 4B is a partial cross-sectional side view of the example medical system of Fig. 4A, showing the snare in the deployed position.
FIG. 5A is a partial cross-sectional side view of the example medical system of Figs. 4A, 4B, showing a distal portion of the electrosurgical device being delivered to a treatment site.
FIG. 5B is a partial cross-sectional side view of the example medical system of Figs. 4A, 4B, showing a target tissue portion of target tissue being withdrawn into a distal chamber.
FIG. 5C is a partial cross-sectional side view of the example medical system of Figs. 4A, 4B, showing a bipolar electrode assembly applying current to a base of the target tissue portion.
FIG. 5D is a partial cross-sectional side view of the example medical system of Figs. 4A, 4B, showing the snare cutting the target tissue portion at the base and partially disposed in a lumen.
FIG. 5E is a partial cross-sectional side view of the example medical system of Figs. 4A, 4B, showing the target tissue portion completely detached from the underlying tissue.
FIG. 6 shows a partial cross-sectional side view of another example medical system that includes an eletrosurgical device having a monopolar electrode assembly.
FIG. 7A is a partial cross-sectional side view of the example medical system of Fig. 1, showing a distal portion of the electrosurgical device being delivered to a treatment site.
FIG. 7B is a partial cross-sectional side view of the example medical system of Fig. 1, showing a bipolar electrode assembly applying current to a bleeding portion of a target tissue portion in order to stop the bleeding.
FIG. 7C is a partial cross-sectional side view of the example medical system of Fig. 1, showing the distal portion of the electrosurgical device being withdrawn from the treatment site and the bleeding portion, having stopped bleeding, retracted back to being part of underlying tissue.
FIG. 8 is an anatomical cross-sectional side view of two layers of a gastrointestinal wall forming a target tissue portion that is withdrawn into a distal chamber of an electrosurgical device.

### DETAILED DESCRIPTION

The present description describes electrosurgical devices and related systems and methods used to maintain a target tissue portion in a raised position from underlying tissue through application of electrical current to a base of the target tissue portion. The target tissue portion may be initially positioned in the raised position through application of a proximal bias, such as a suction. Applying the electrical current to the base may create a scarring of the tissue may occur at the base, which may maintain the target tissue portion in the raised position when the proximal bias is removed.

Rather than apply the electrical current the base, other devices may deploy a band around the base to maintain the target tissue portion in the raised position. Through application of electrical current rather than deploying a band, the electrosurgical device is not limited by the number of bands that can loaded onto the device at a single device when creating numerous raised target tissue portions at a treatment site.

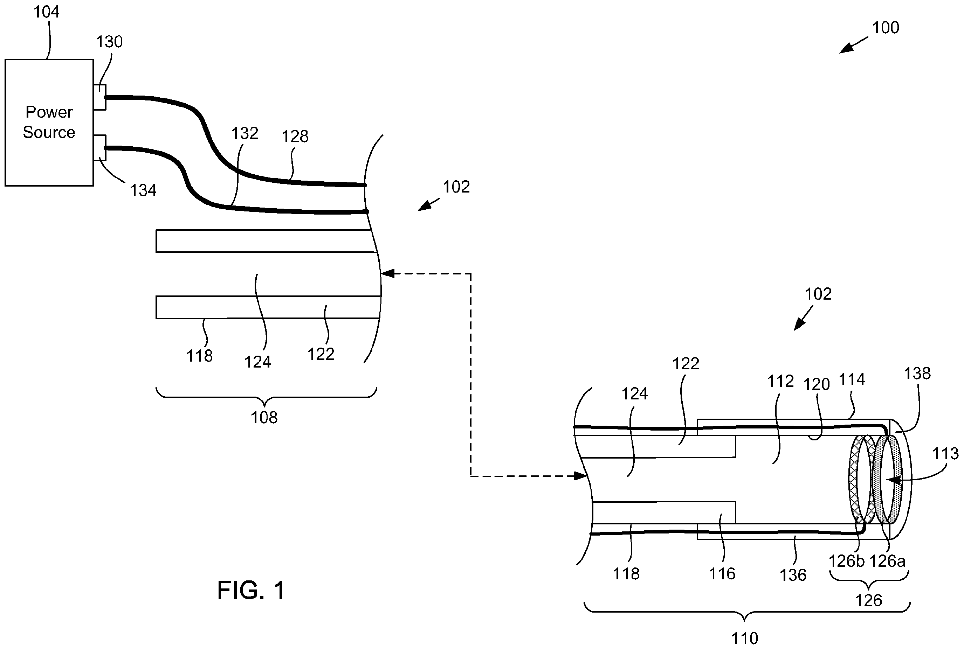

Fig. 1 shows a partial cross-sectional side view of an example electrosurgical medical system 100 that includes an elongate tubular electrosurgical medial device 102 coupled to each of a power source 104 and a suction source 106. The electrosurgical device 102 may longitudinally extend from a proximal portion 108 to a distal portion 110. The distal portion 110 may include a distal chamber 112 having a distal opening 113 for withdrawal of tissue therein. For some example configurations, as the one shown in Fig. 1, the elongate tubular electrosurgical device 102 may include an elongate tubular structure that comprises a first elongate tubular member 114, such as a cap, disposed about and affixed to a distal end 116 of a substantially longer second elongate tubular member 118, such as an endoscope or a catheter as examples. The first tubular member 114 may have an inner surface 120 that defines the distal chamber 112. The second tubular member 118 may include a body 122 and a suction lumen 124 longitudinally extending in the body 122 from the proximal portion 108 to the distal portion and in fluid communication with the distal chamber 112. For other example configurations, the elongate tubular structure may be an integral structure in which the distal chamber 112 is a distal-most portion of the suction lumen 124.

The electrosurgical device 102 may further include an electrode assembly 126 disposed in the distal chamber 112. The electrode assembly 126 may include at least one electrode electrically coupled to the power source 104. In the example configuration shown in Fig. 1, the electrode assembly 126 includes two electrodes 126a, 126b configured in a bipolar configuration. In accordance with the bipolar configuration, one of the electrodes 126a is an active electrode that is part of a conductive active path that further includes an active conductive wire, cable, or other elongate conductive member 128 that electrically couples the active electrode 126a to an active port 130 of the power source 104. The other electrode 126b is a return electrode that is part of a conductive return path that further includes a return conductive wire, cable, or other elongate conductive member 132 that electrically couples the return electrode 126b to a return port 134 of the power source 104.

The electrode assembly 126 in the bipolar configuration may differ from an electrode assembly in a monopolar assembly in that, in accordance with the bipolar configuration, the return path may be attached to, adhered to, integrated with, disposed within, extend alongside, or included as part of the tubular members 112, 118. In contrast, under a monopolar configuration, a neutral electrode (e.g., a solid, neutral electrode or a split neutral electrode) positioned on the patient may be used for the return path.

In the example bipolar configuration shown in Fig. 1, each of the active and return wires 128, 132 may distally extend from respective ports 130, 134 outside of and alongside the second elongate tubular member 118 to within a wall 136 of the first tubular member 114 at the distal portion 110, where then each extend and connect to and a respective one of the electrodes 126a, 126b at the inner surface 120. For other example configurations, the active and return wires 128, 132 may extend within the second tubular member 118, such as within the suction port 124, back to the ports 130, 134 of the power source 104.

The electrodes 126a, 126b may each be ring-shaped structures circumferentially disposed on the inner surface 120. In some example configurations, such as shown in Fig. 1, the electrodes 126a, 126b may be continuous ring-shaped structures such that they are each completely circumferentially disposed on the inner surface 120. Alternatively, the electrodes 126a, 126b may be discontinuous ring-shaped structures such that they are each only partially circumferentially disposed on the inner surface 120. Additionally, the electrodes 126a, 126b may be oriented in planes substantially transverse to the longitudinal axis in which the electrosurgical device 102 longitudinally extends. Also, for the example configuration shown in Fig. 1, the active electrode 126a is positioned distal the return electrode 126b, although the reverse configuration may be used for other example configurations.

The electrode assembly 126 may be longitudinally positioned at a distal portion at or near the distal opening 113 of the distal chamber 112. For some example configurations, the distal-most electrode of the assembly 126 (e.g., the active electrode 126a shown in Fig. 1), may be flush with a distal end 138 of the first elongate member 114. As described in further detail below, a target tissue portion of underlying tissue may be withdrawn into the distal chamber 112. The electrode assembly 126 may contact the target tissue portion. In addition, the electrode assembly 126, upon receipt of radio frequency (RF) electrical current from the power source 104, may scar the target tissue portion.

Where the target tissue portion is scarred and the size and shape of the area of the target tissue portion that is scarred may be depend on where the electrodes 126a, 126b contact the target tissue portion in the distal chamber 112 and how far apart the electrodes 126a, 126b are separated from each other. Longitudinally, scarring may occur in between the electrodes 126a, 126b and so a thickness of the scarring area may correspond to a longitudinal spacing in between the proximal electrode 126b to the distal electrode 126a. Circumferentially, the scarring area may correspond to the circumferential shape of the ring-shaped electrodes 126a, 126b. Where the electrodes 126a, 126b are continuous ring-shaped structures and are completely circumferentially disposed on the inner surface 120, then scarring may circumferentially extend completely around the target tissue portion. Alternatively, where the electrodes 126a, 126b are discontinuous ring-shaped structures such that they are not completely circumferentially disposed on the inner surface 120, then the extent to which scarring circumferentially occurs may correspond to the extent to which the discontinuous ring-shaped structures circumferentially extend on the inner surface 120. As an example, where the ring-shaped electrodes 126a, 126b are semi-circular structures that circumferentially extend about 80% of the total circumference of the inner surface 120, then scarring may circumferentially extend about 80% of the target tissue portion.

By being disposed in the distal portion of the distal chamber 112, the electrode assembly 126 may scar a distal area of the target tissue portion. The distal area of the target tissue portion may be scarred so that the target tissue portion is maintained in a raised position relative to the underlying tissue when no other forces (e.g., suction) are acting on the target tissue portion.

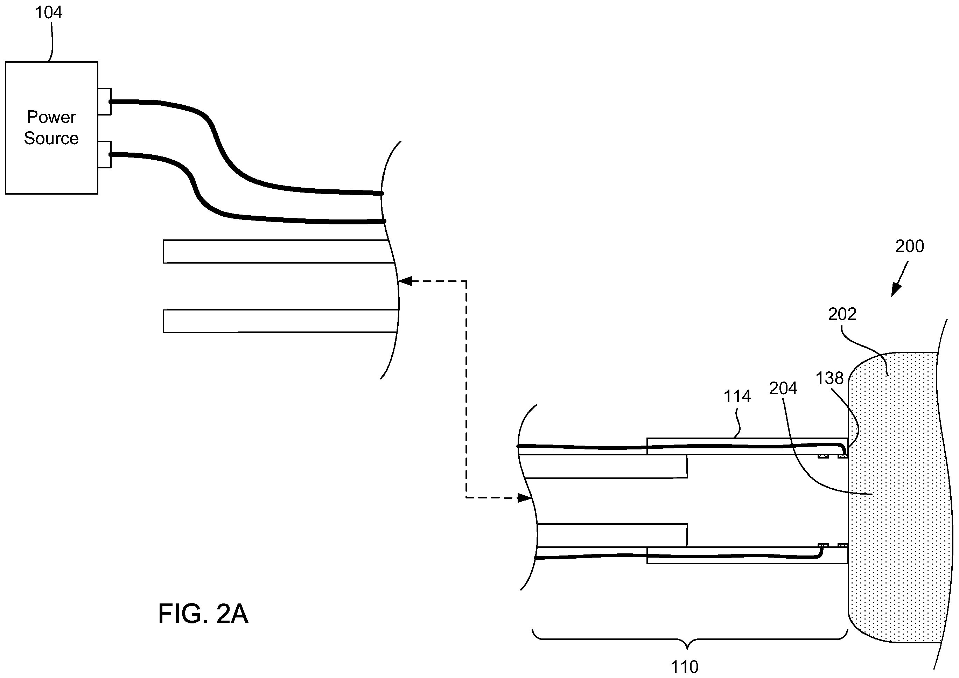

A method of raising and resecting a target tissue portion using the example medical system 100 is described with reference to Figs. 2A-2G. Referring to Fig. 2A, the distal portion 110 may delivered to a treatment site 200. An example treatment site may be an area in the gastrointestinal tract within a patient, such as the stomach or the esophagus, although other biological treatment sites may be possible. The treatment site 200 may include underlying tissue 202 from which a target tissue portion 204 is to be resected or removed. As shown in Fig. 2A, the distal end 138 of the first tubular member 114 may be positioned adjacent the target tissue portion 204 so that the target tissue portion 204 may be withdrawn into the distal chamber 112.

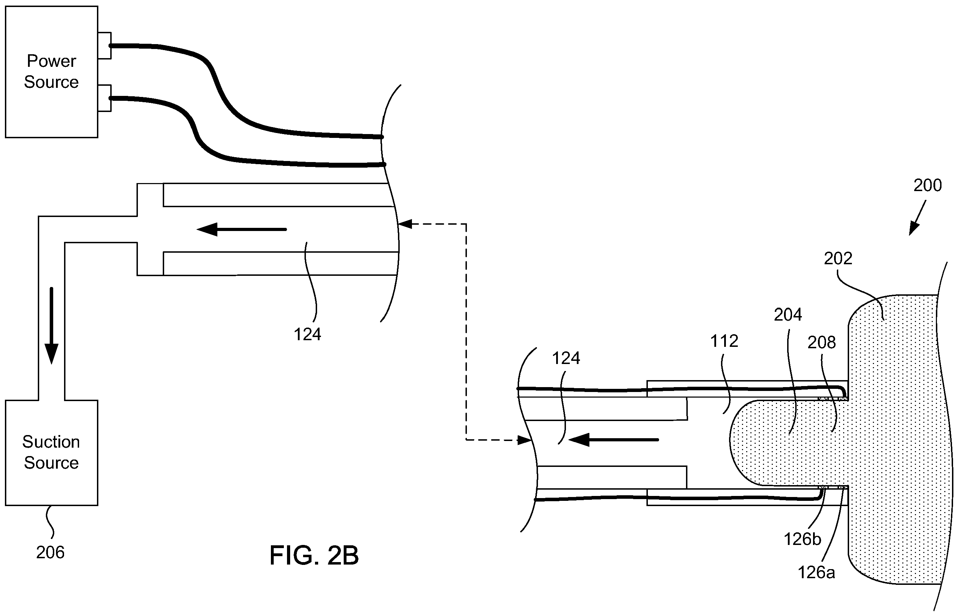

Referring to Fig. 2B, a proximal bias may be applied to the target tissue portion 204, which may withdraw the target tissue portion 204 into the distal chamber 112. For some example methods, the proximal bias may be a suction. For example, as shown in Fig. 2B, a suction source 206 may be connected to the suction lumen 124. Upon activation of the suction source 206, a suction may be applied to the suction lumen 124, which in turn proximally biases the target tissue portion 204, withdrawing the target tissue portion into the distal chamber 112. As the target tissue is withdrawn into the distal chamber 112, it may pass through the active and return electrodes 126a, 126b. For other example methods, a proximal bias other than a suction may be used. For example, an anchoring or traction mechanism may penetrate the target tissue portion 204 and mechanically pull the target tissue portion 204 into the distal chamber 112.

While the proximal bias is being applied and the target tissue portion 204 is in the distal chamber 112, the target tissue portion 204 is in a raised position in that it is raised above or away from the underlying tissue 202. When the target tissue portion is in the distal chamber 112, the electrodes 126a, 126b may contact a distal or base portion 208 of the target tissue portion 204.

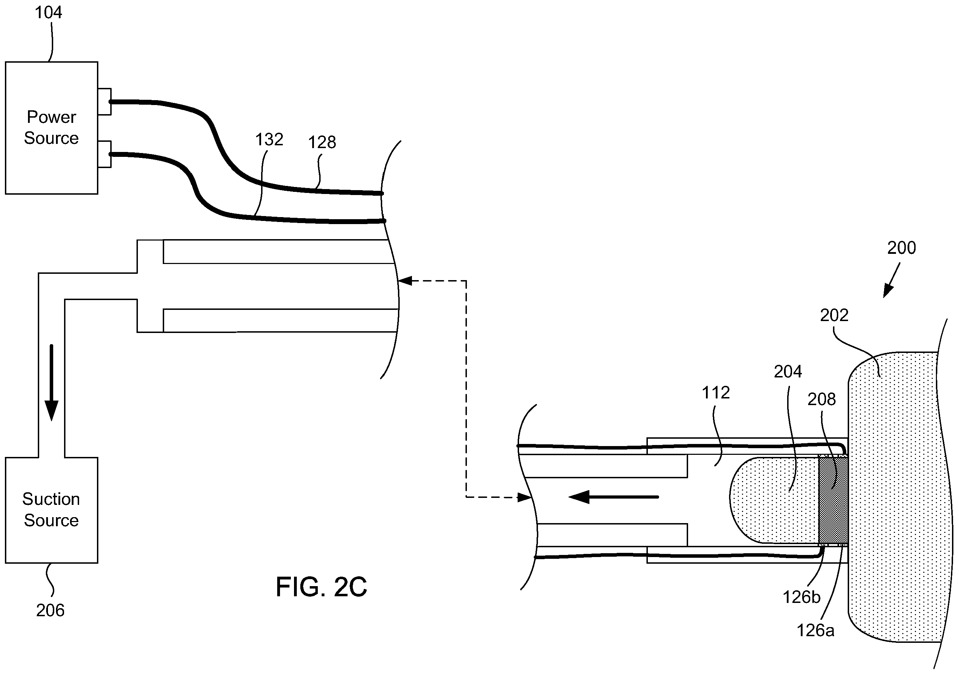

Referring to Fig. 2C, while the proximal bias is applied and the target tissue portion 204 is in the raised position in the distal chamber 112, electrical current may be delivered to the electrode assembly 126. In particular, electrical current may be delivered from the power source 104, through the active wiring or cabling 128 and the active electrode 126a of the active path, and applied to the base portion 208 of the target tissue portion 204. After passing through the base portion 208, the electrical current may then return back to the power source 104 via the return electrode 126b and the return wiring or cabling 132 of the return path.

The electrical current applied to the base portion 208 may coagulate the base portion 208, which may result in a scarring of the base portion 208 (denoted by a darkened shading of the base portion 208 in Fig. 2C). The area that is scarred may depend on where the electrodes 126a, 126b contact the base portion 208 and the spacing in between the electrodes 126a, 126b, as previously described. The proximal bias and the electrical current may be applied until the base portion 208 has undergone a sufficient amount of scarring. A sufficient amount of scarring may occur when the target tissue portion is maintained in the raised position without being proximally biased, rather than recessing back into the underlying tissue 202.

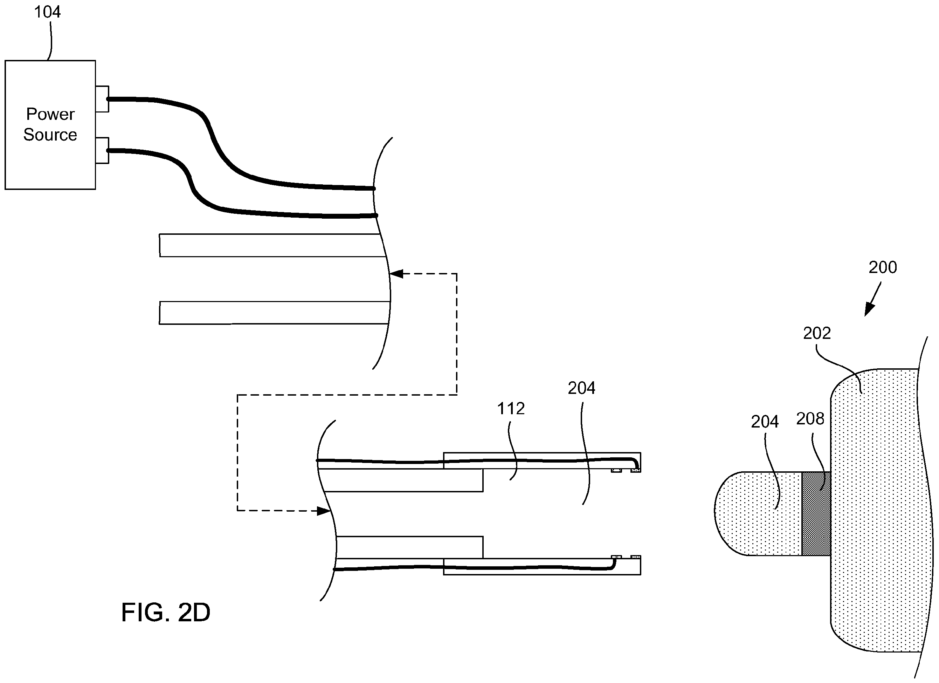

Referring to Fig. 2D, when the base portion has sufficiently scarred, application of the proximal bias and the electrical current may be removed. Despite the target tissue portion no longer being subjected to the proximal bias, it may be maintained in the raised position. By scarring the base portion 208 rather than applying a band around the base portion 208 to maintain the target tissue portion 204 in the raised position, the number of times that the electrosurgical device 102 can create raised polyps at the treatment site 200 may not be limited by the number of bands that are allowed to be loaded onto the device at a single time, as is the case with multi-band mucosectomy devices.

After the base portion 208 has undergone sufficient scarring and the target tissue portion 204 is maintained in the raised position, the target tissue portion 204 may be resected or detached from the underlying tissue 202. For some example methods, an electrosurgical snare 210 may be used to resect the target tissue portion 204, although other example methods may use other medical detachment devices to remove the target tissue portion 204.

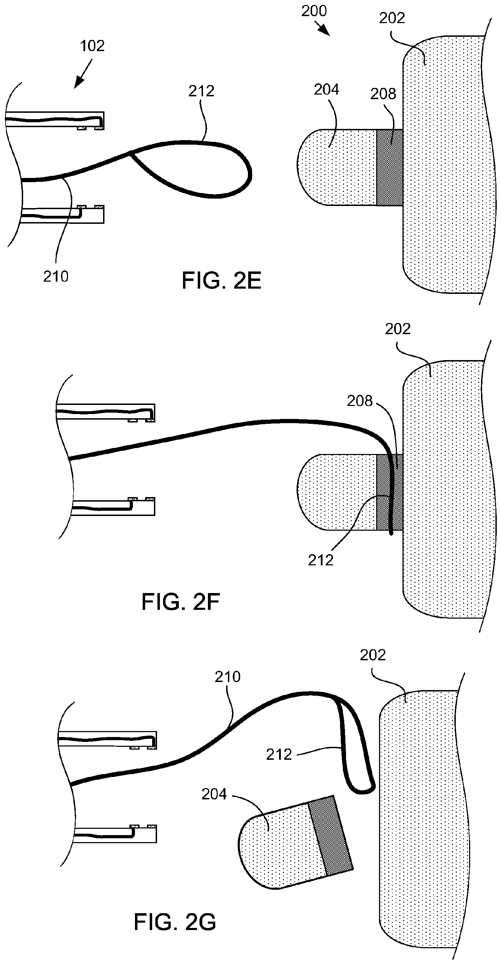

Referring to Fig. 2E, a distal loop portion 212 of the snare 210 may be delivered to the treatment site 200. The distal loop portion 212 may delivered through the electrosurgical device 102 to reach the treatment site 200. Referring to Fig. 2F, the distal loop portion 212 may be positioned around the scarred base portion 208. Referring to Fig. 2G, the electrical current may be delivered to the snare 210, and the distal loop portion 212, upon receiving the electrical current, may cut the base portion 208 until the target tissue portion 204 is detached from the underlying tissue 202.

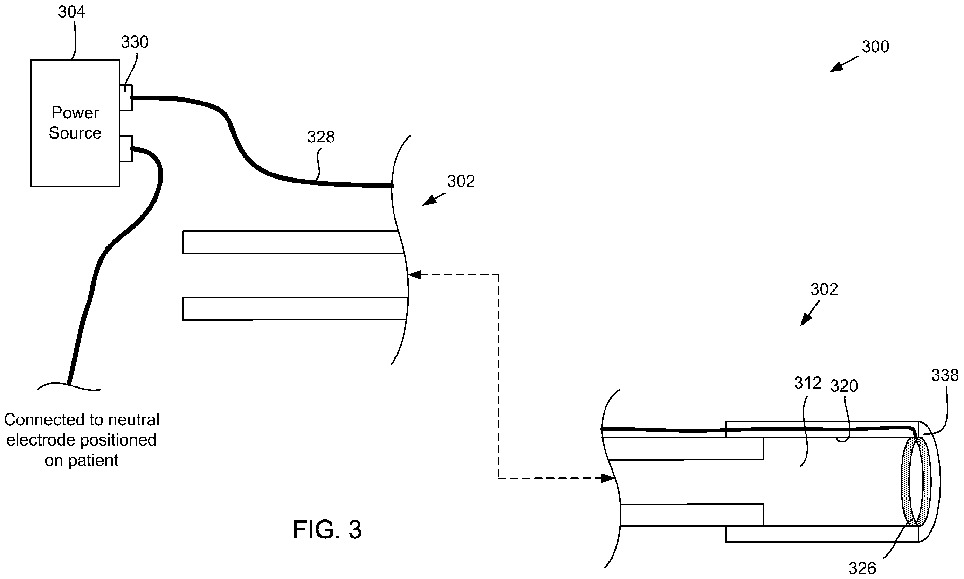

Fig. 3 shows a partial cross-sectional side view of another example medical system 300 that includes an elongate tubular electrosurgical medical device 302 coupled to a power source 304. The electrosurgical device 302 may be similar to the electrosurgical device 102 shown and described with reference to Figs. 1 and 2A-2F, except that instead of having an electrode assembly configured in a bipolar configuration, the electrosurgical device 302 may include an electrode assembly 326 configured in a monopolar configuration. As shown in Fig. 3, the electrode assembly 326 may include a single ring-shaped electrode 326 disposed on an inner surface 320 of a distal chamber 312 at or near a distal end 338 of the electrosurgical device 302. The electrode 326 may be part of an active path that also includes an active wiring, cabling or elongate conductive member 328 that electrically couples the active electrode 326 to an active port 330 of the power source 304. Also, rather than have a return path that includes a return electrode disposed in the distal chamber 312 and return wiring extending alongside the electrosurgical device 302, the return path for the example medical system 300 may include a neutral electrode (not shown) positioned on the patient.

The example medical system 300 may raise a target tissue portion and scar a base portion of the target tissue portion in the same way as does the example medical system 100 shown and described with reference to Figs. 1 and 2A-2F, except the thickness of the target tissue portion that is scarred is determined by the single electrode 326 rather than two electrodes 126a, 126b and the spacing between them.

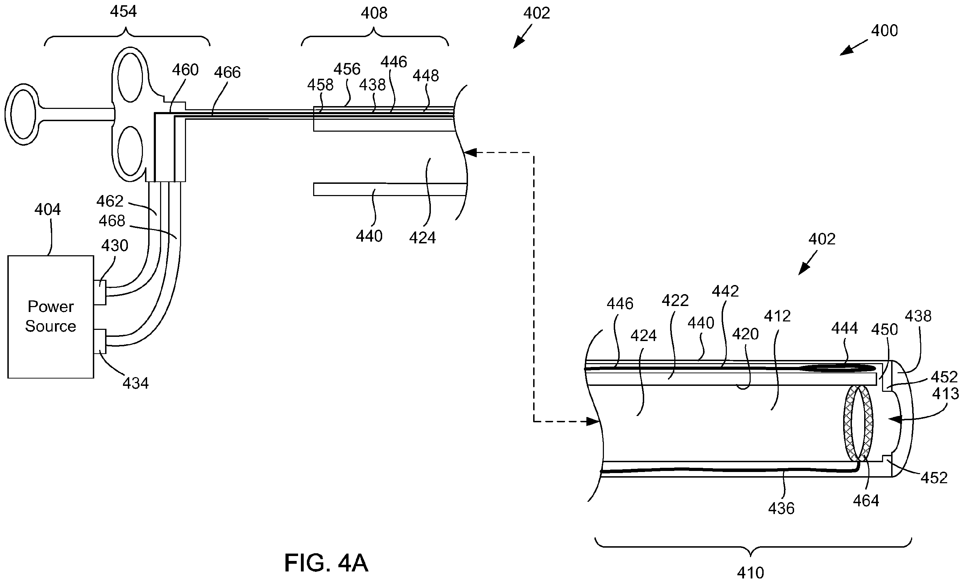

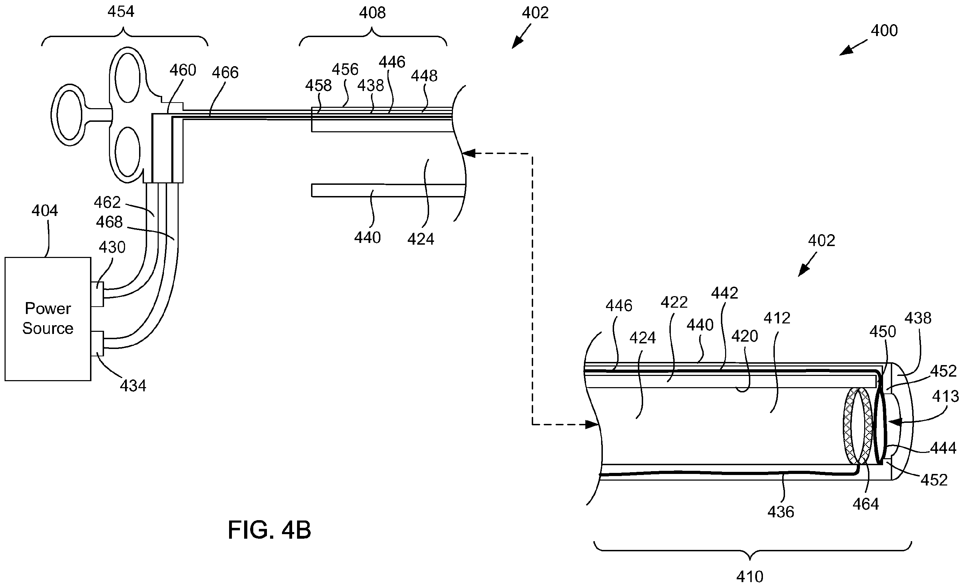

Figs. 4A and 4B shows a partial cross-sectional side view of another example medical system 400 that includes an elongate tubular electrosurgical device 402 longitudinally extending from a proximal portion 408 to a distal portion 410. The electrosurgical device 402 may include an elongate tubular member 440 that longitudinally extends from the proximal portion 408 to the distal portion 410. For some example configurations, as shown in Figs. 4A and 4B, the elongate tubular member 440 may be an integral structure. For other example configurations, the elongate tubular member 440 may include a first elongate tubular member, such as a cap, attached to a distal end of a second elongate tubular member, such as an endoscope or a catheter, similar to the example configurations shown and described with reference to Figs. 1, 2A-2F, and 3. The elongate tubular member 440 may include a distal chamber 412 disposed at the distal portion 410 and defined by an inner surface 420 of a body 422 of the tubular member 440. The tubular member 440 may also include a suction lumen 424 longitudinally extending in the body 422 from the proximal portion 408 to the distal portion 410, and in fluid communication with the distal chamber 412.

The example medical system 400 may further include an electrosurgical snare 442 that includes a distal loop portion 444 and a proximal stem portion 446 connected to the distal loop portion 444. The electrosurgical snare 442 may be longitudinally and movably disposed in a snare lumen 448 that longitudinally extends in the body 422 from the proximal portion 408 to the distal portion.

The distal loop portion 444 may be movable between an undeployed position and a deployed position. Fig. 4A shows the distal loop portion 444 in the undeployed position. Fig. 4B shows the distal loop portion 444 in the deployed position. As shown in Fig. 4A, in the undeployed position, the distal loop portion 444 may be at least partially disposed in the snare lumen 448. In the deployed position, as shown in Fig. 4B, the distal loop portion 444 may be disposed in the distal chamber 412. When disposed in the distal chamber, the distal loop portion 444 may be circumferentially disposed adjacent the inner surface 420 defining the distal chamber 412 and in a plane that is substantially transverse to the longitudinal axis in which the electrosurgical device 402 longitudinally extends.

The elongate tubular member 440 may include a port 450 extending between the snare lumen 448 and the distal chamber 412. The distal loop portion 444 may move through the port 450 to move between the undeployed and the deployed positions. In addition, the elongate tubular member 440 may include a lip or shoulder 452 disposed at a distal end 438 of the tubular member 440. The lip or shoulder 452 may cause a diameter of a distal opening 413 of the tubular member 440 to be smaller than an inner diameter of the distal chamber 412 defined by the inner surface 420 such that, as shown in Fig. 4B, when the distal loop portion 444 of the snare 442 is in the deployed position, it may rest or press up again the lip or shoulder 452. This may prevent the distal loop portion 444 from slipping through the distal opening 413 to outside the distal chamber 412 when in the deployed position.

The bipolar sphincterotome 102 may further include a handle assembly 454 coupled to a proximal end 458 of the tubular member 106. The handle assembly 130 may be operatively coupled to a proximal end 458 of the proximal stem portion of the snare 442, and configured to move the distal loop portion between the deployed and undeployed positions. Figs. 4A and 4B show the handle assembly 454 being configured as a three-ringed structure for gripping by an operator of the electrosurgical device 402, although other configurations for the handle assembly 454 may be possible.

The distal loop portion 444 of the snare 442 may be an active electrode of a bipolar electrode assembly of the electrosurgical device 402. As the active electrode, the distal loop portion 444 may be part of an active path of the electrosurgical device 402 that further includes the proximal stem portion 446 of the snare 442. For the example configuration shown in Figs. 4A and 4B, the snare 442 may be electrically coupled to an active port 430 of a power source 404 via an active coupling portion 460 and an active power cord 462 of the handle assembly 454.

The bipolar electrode assembly may further include a return electrode 464 disposed on the inner surface 420 in the inner chamber. The return electrode 464 may be a conductive ring-shaped structure, similar to the ring-shaped return electrode 126b shown and described with reference to Figs. 1 and 2A-2F. The return electrode 464 may be positioned in the distal chamber 412 in a plane that is substantially transverse to the longitudinal axis of the electrosurgical device 402 such that when the distal loop portion 444 is in the deployed position as shown in Fig. 4B, the distal loop portion 444 and the return electrode 464 may be substantially parallel with each other.

The return electrode 464 may be part of a return path of the electrosurgical device 402, that further includes conductive return wiring 436 used to electrically couple the return electrode 464 with a return port 434 of the power source 404. The return wiring 436 may be configured with the body 422 of the tubular member 440 in various ways. For some example configurations, as shown in Figs. 4A and 4B, the return wiring 436 may longitudinally extend in the tubular member 440 outside of the snare lumen 448 at the distal portion 410 and transition into the snare lumen 448 at the proximal portion 408, where the return wiring 436 may extend alongside the stem portion 446 of the snare 442. The handle assembly 454 may further include a return coupling portion 466 and a return power cord 468 that electrically couples the return wiring 436 to the return port 434. For other example configurations, the return wiring 436 may extend in the snare lumen 448 from the proximal portion 408 to the distal portion 410. In still other example configurations, the return wiring 436 may extend completely outside of the snare lumen 448, either within or outside of and alongside the body 422 of the tubular member 440, and/or may or may not be coupled to the return port 434 via the handle assembly 454.

In addition, as shown in Fig. 1, the proximal end 134 of the active conductive member 112 and a proximal end 136 of the unexposed member 128 may each be electrically coupled to the power source 104 via the handle assembly 130, although alternative configurations may be possible. An active power cord 138 may be connected to an active port 140 of the power source 104 and to the handle assembly 130 to electrically couple the active port 130 of the power source 104 with the active conductive member 112. Similarly, a return power cord 142 may be connected to a return port 144 of the power source 104 and to the handle assembly 130 to electrically couple the unexposed member 128 of the return path to the return portion 144 of the power source 104.

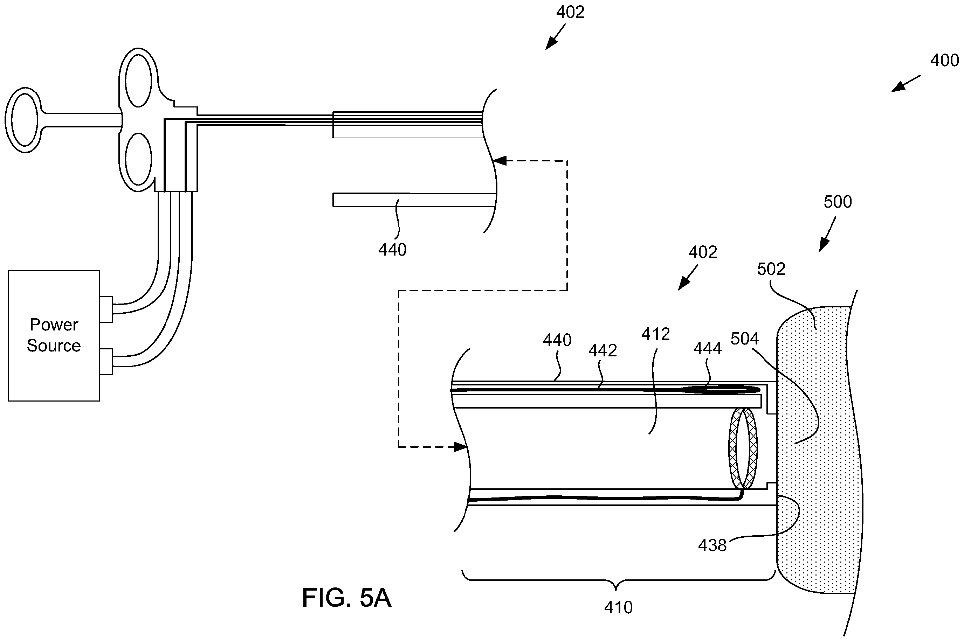

A method of raising and resecting a target tissue portion using the example medical system 400 is described with reference to Figs. 5A-5D. Referring to Fig. 5A, the distal portion 410 of the electrosurgical device 402 may delivered to a treatment site 500, such as an area in a gastrointestinal tract or other biological area of a patient. The treatment site 500 may include underlying tissue 502 from which a target tissue portion 504 is to be resected or removed. As shown in Fig. 5A, the distal end 438 of the tubular member 440 may be positioned adjacent the target tissue portion 504 so that the target tissue portion 504 may be withdrawn into the distal chamber 412.

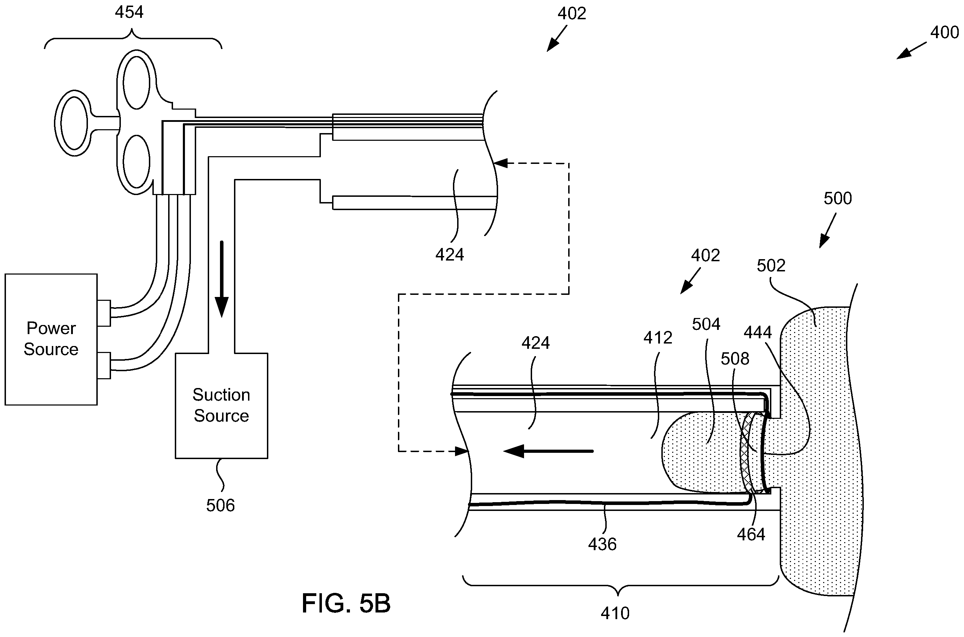

The distal portion 410 may be delivered to the treatment 500 with the distal loop portion 444 of the snare 442 in either the undeployed position or the deployed position. Referring to Fig. 5B, if the distal loop portion 444 is in the undeployed position when the distal portion 410 is delivered to the treatment site 500, then the distal loop portion 444 may be moved to the deployed position in the distal chamber 412, such as by using the handle assembly 454. After the distal loop portion 444 is deployed into the distal chamber 412, a proximal bias may be applied to the target tissue portion 504, which may withdraw the target tissue portion 504 into the distal chamber 412. For some example methods, the proximal bias may be suction. For example, as shown in Fig. 5B, a suction source 506 may be connected to the suction lumen 424. Upon activation of the suction source 506, a suction may be applied to the suction lumen 424, which in turn proximally biases the target tissue portion 504, withdrawing the target tissue portion into the distal chamber 412. For other example methods, a proximal bias other than a suction may be used. For example, an anchoring or traction mechanism may penetrate the target tissue portion 504 and mechanically pull the target tissue portion 504 into the distal chamber 412.

As shown in Fig. 5B, when the target tissue portion 504 is withdrawn into the distal chamber 412, the target tissue portion 514 may pass through the distal loop portion 444 in the deployed position 444. While the proximal bias is being applied and the target tissue portion 504 is in the distal chamber 412, the target tissue portion 504 is in a raised position. When the target tissue portion is in the raised position in distal chamber 412, the distal loop portion 444 and the return electrode 464 may contact a distal or base portion 508 of the target tissue portion 504.

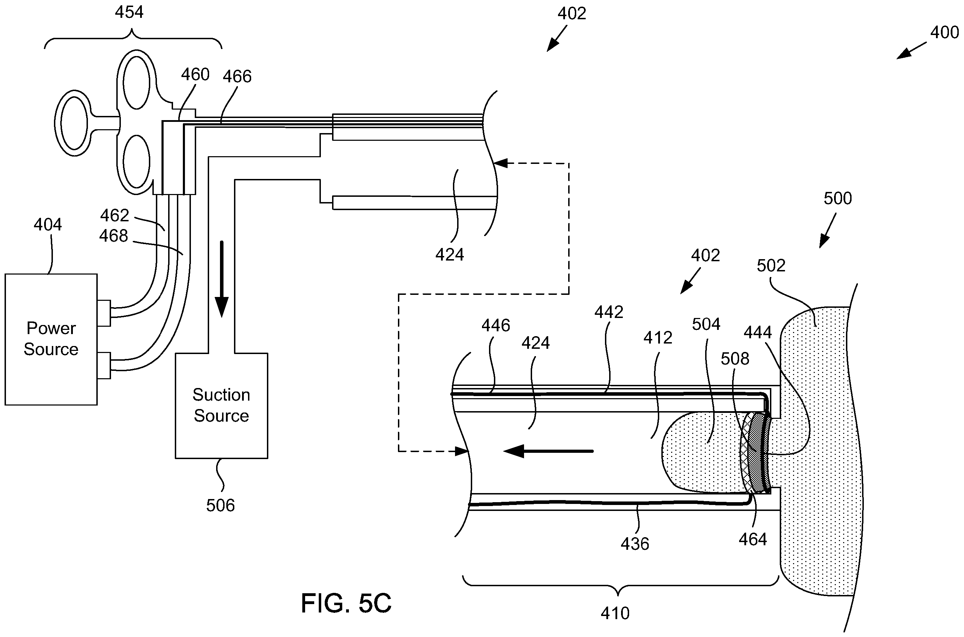

Referring to Fig. 5C, while the proximal bias is applied and the target tissue portion 504 is in the raised position within the distal chamber 412, electrical current may be delivered to the electrode assembly. In particular, electrical current may be delivered from the power source 404 along the active path, including through the active power cord 462 and active coupling portion 460 of the handle assembly 454 and the stem portion 446 and the distal loop portion 444 of the snare 442, and applied to the base portion 508. After passing through the base portion 508, the electrical current may then return back to the power source 404 via the return path, including through the return electrode 464, the return wiring 436, and the return coupling portion 466 and the return power cord 468 of the hand assembly 454.

The electrical current applied to the base portion 508 may coagulate the base portion 508, which may result in a scarring of the base portion 508. The area that is scarred may depend on where the distal loop portion 444 and the return electrode 464 contact the base portion 508 and the spacing in between the distal loop portion 444 and the return electrode 464. The proximal bias and the electrical current may be applied until the base portion 508 has undergone a sufficient amount of scarring such that the target tissue portion 506 may be maintained in the raised position without being proximally biased.

When the base portion has sufficiently scarred, application of the proximal bias and the electrical current may be removed. Despite the target tissue portion no longer being subjected to the proximal bias, it may be maintained in the raised position.

After the base portion 508 has undergone sufficient scarring and the target tissue portion 504 is maintained in the raised position, the target tissue portion 504 may be resected or detached from the underlying tissue 502. For some example methods, the electrosurgical snare 442 used to create the scarring area 508 may also be used to resect the target tissue portion 504, although other example methods may use other medical devices to remove the target tissue portion 504.

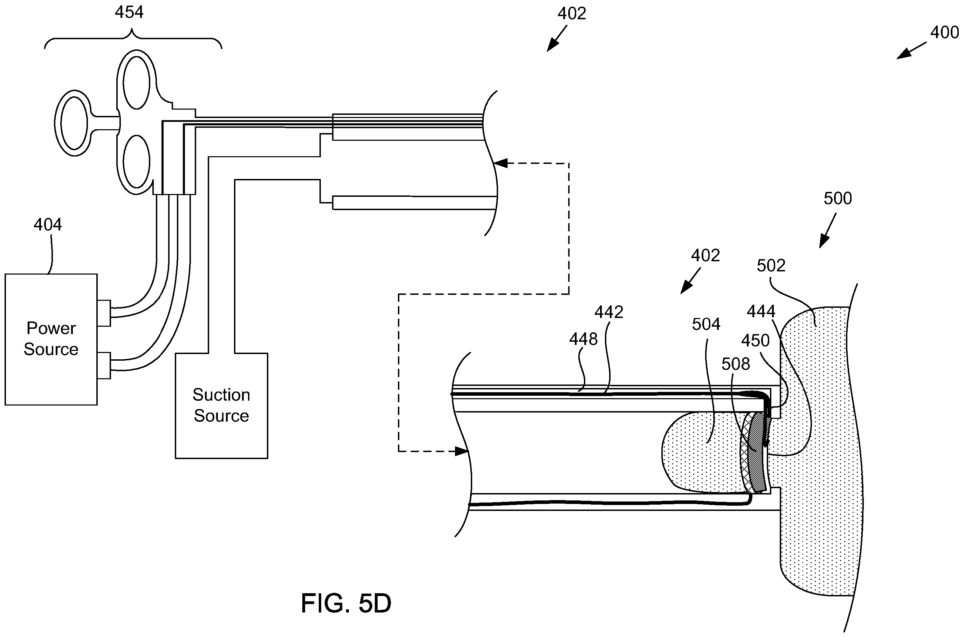

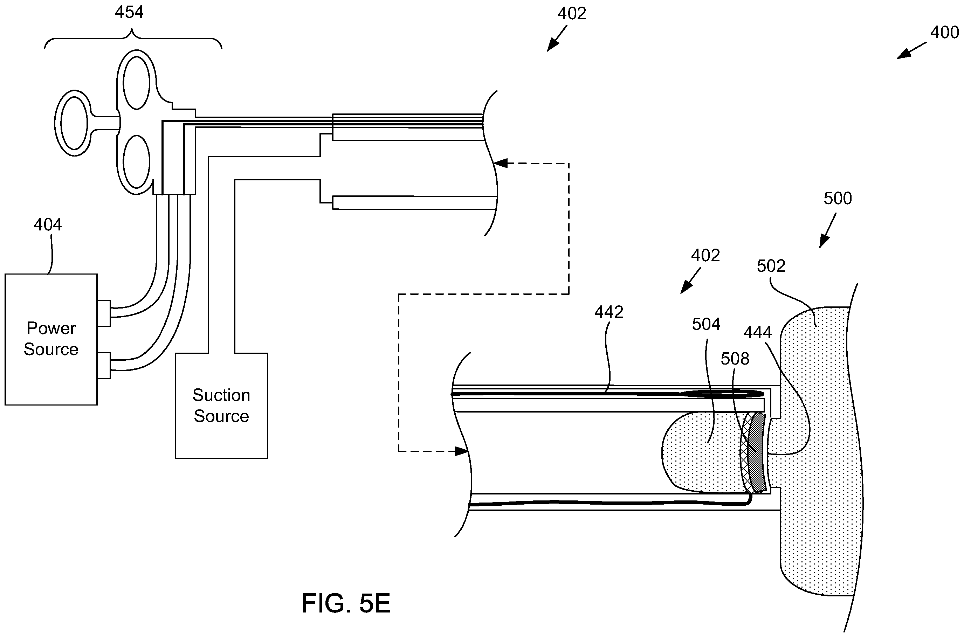

Referring to Figs. 5D and 5E, a different power and frequency setting may be set on the power source 404 for the electrical current being delivered to the distal loop portion 444 so that the distal loop portion 444 cuts the target tissue portion rather than coagulates it. After the power and frequency settings are adjusted, electrical current at the adjusted settings may be delivered to the distal loop portion 444 and applied to the base portion 508. While the distal loop portion 444 applies the electrical current to the base portion 508, the distal loop portion 444 may be moved, such as by using the handle assembly 454, through the port 450 back to the undeployed position in the snare lumen 448 to cut the target tissue portion 504 at the scarred base 508. Fig. 5D shows the distal loop portion 444 partially withdrawn back into the snare lumen 448, with the target base portion 508 being partially cut. Fig. 5E shows the distal loop portion 444 completely withdrawn back into the snare lumen 448, with the target tissue portion 504 being completely detached from the underlying tissue 502.

For other example methods, the distal loop portion 444 may cut the target tissue portion 504 while in the raised position without first scarring the base portion 508. However, scarring the base portion 508 first and then cutting the base portion 508 may reduce the amount of bleeding that occurs at the treatment site.

Additionally, for the example method described above, the proximal bias, such as the suction, may be removed once sufficient scarring at the base portion 508 has occurred and prior to detaching the target tissue portion 504 from the underlying tissue 502. For other example methods, the proximal bias may be maintained while the distal loop portion 444 cuts the base portion 508, which may facilitate the cutting.

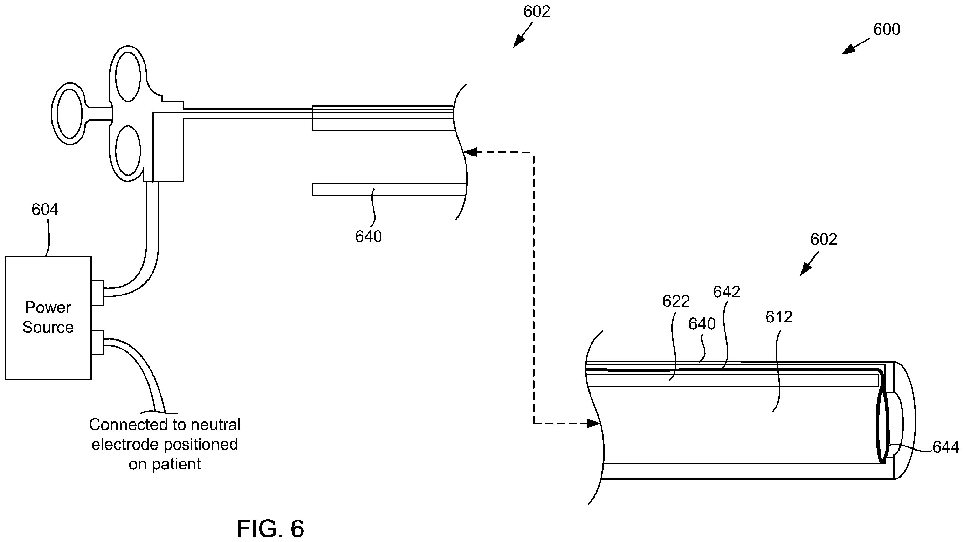

Fig. 6 shows a partial cross-sectional side view of another example medical system 600 that includes an elongate tubular electrosurgical medical device 602 coupled to a power source 604. The electrosurgical device 602 may be similar to the electrosurgical device 402 shown and described with reference to Figs. 4 and 5A-5E, except that instead of having a distal loop portion of an electrosurgical snare implemented as an active electrode for a bipolar electrode assembly, a distal loop portion 644 movable of an electrosurgical snare 642 movable between undeployed and deployed positions may be an active electrode for a monopolar electrode assembly. For the monopolar configuration, rather than have a return path that includes a return electrode disposed in a distal chamber 612 and return wiring longitudinally extending within and/or alongside a body 622 of an elongate tubular member 640, the return path for the example medical system 600 may include a neutral electrode (not shown) positioned on the patient.

The example medical system 600 may raise a target tissue portion, scar a base portion, and detach the target tissue portion from underlying tissue in the same way as does the example medical system 400 shown and described with reference to Figs. 4A, 4B, and 5A-5E, except the thickness of the target tissue portion that is scarred may be determined by the single, distal loop portion 644 rather than the combination of the distal loop portion and a return electrode.

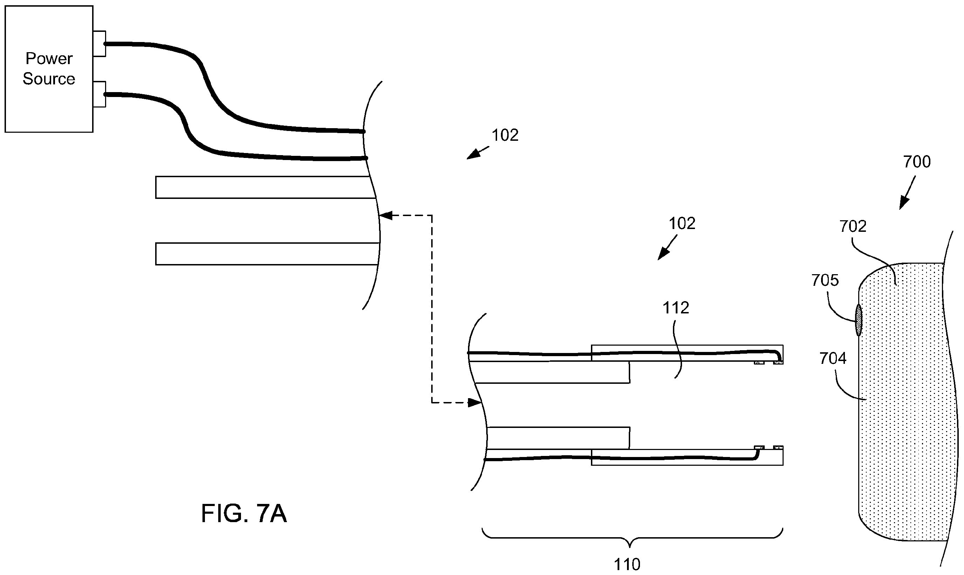

In addition or alternatively to raising a target tissue portion and scarring a base portion, the electrosurgical medical systems 100 and 300 of Figs. 1 and 3, respectively, may be configured to perform hemostasis, that is, stop bleeding tissue. An example method of performing hemostasis is described with reference to Figs. 7A-7C. The method described with reference to Fig. 7A-7C is described as being performed with the electrosurgical medical system 100 of Fig. 1. However, the medical system 300 of Fig. 3 may similarly be used to perform the method.

Referring to Fig. 7A, the distal portion 110 of the electrosurgical device 102 may be delivered to a treatment site 700, which as before, may be an area in the gastrointestinal tract within a patient, such as the stomach or the esophagus, although other biological treatment sites may be possible. At the treatment site 700, underlying tissue 702 may include a target tissue portion 704 to be withdrawn into the distal chamber 112, at least a portion 705 of which may be bleeding.

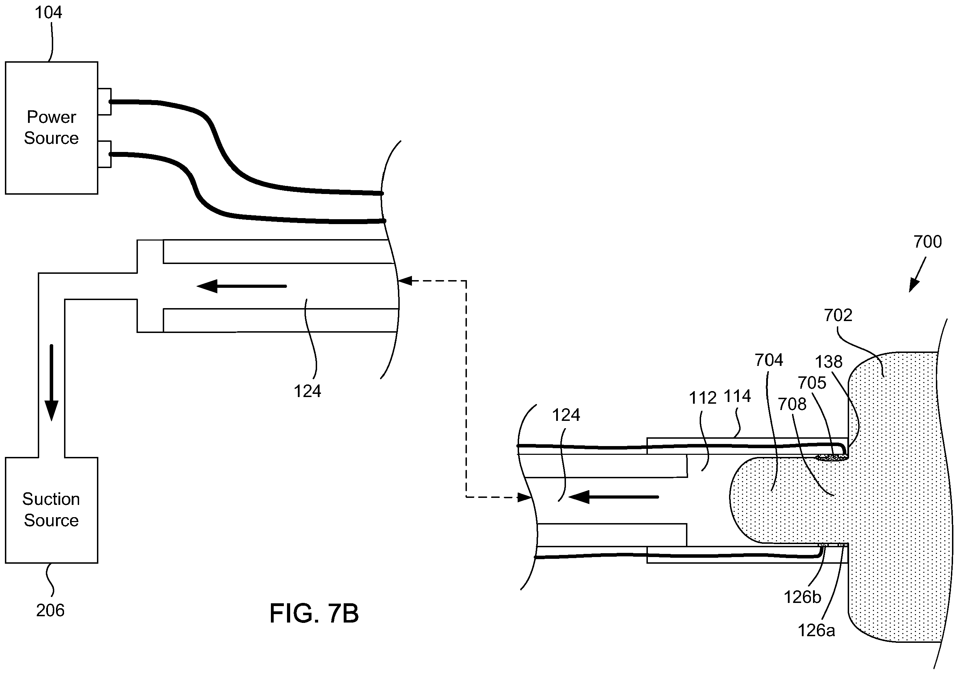

Referring to Fig. 7B, the distal end 138 of the first tubular member 114 may be positioned adjacent the underlying tissue 702, and the vacuum source 206 may be activated in order to apply a suction to the lumen 124 and withdraw the target tissue portion 704 into the distal chamber 112.

Upon activation of the suction source 206, a suction may be applied to the suction lumen 124, which in turn proximally biases the target tissue portion 704, withdrawing the target tissue portion 704 into the distal chamber 112. Prior to applying the suction, the distal end 138 may be positioned at a location relative to the bleeding portion 705 so that when suction is applied and the target tissue portion 704 and is withdrawn into the distal chamber 112, the bleeding portion 705 is in contact with, longitudinally in between the electrodes 126a, 126b, and/or otherwise at a location within the chamber 112 relative to the electrodes 126a, 126b so that, upon electrical activation, the electrodes 126a, 126b coagulate the bleeding portion 705. The target tissue portion 704 may be part of a base portion 708 of the target tissue portion 704. Additionally, the spacing in between the electrodes 126a, 126b may vary for different configurations of the electrosurgical device 102. The larger the spacing, the larger the amount of bleeding tissue the device 102 may coagulate.

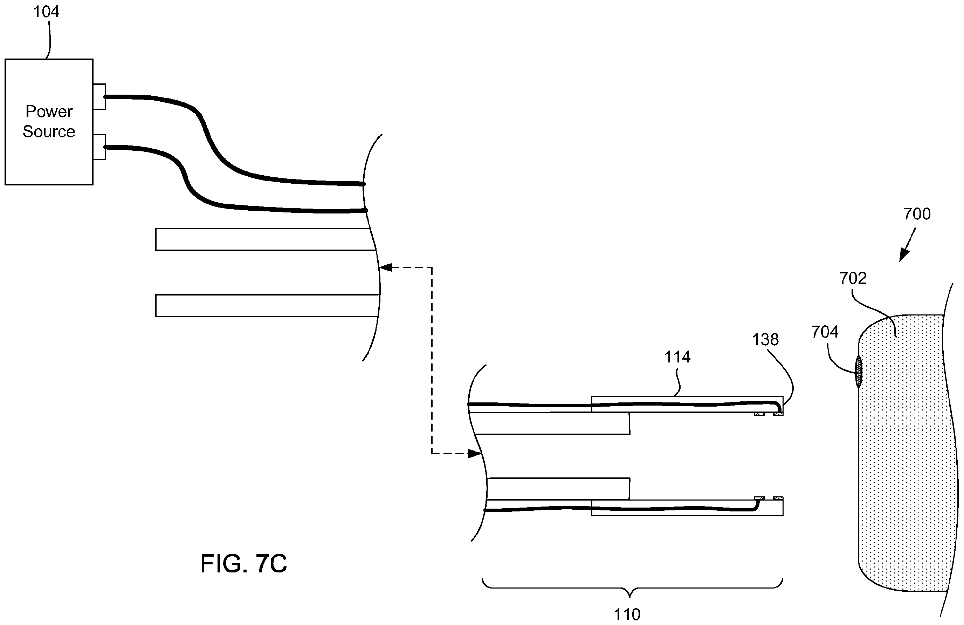

After the suction is applied to the lumen 124 and the bleeding portion 705 is at a desired location within the chamber 112 relative to the electrodes 126a, 126b, the power source 104 may be activated, sending electrical current to the electrodes 126a, 126b, which in turn may coagulate the bleeding portion 705 to stop the bleeding. Referring to Fig. 7C, after a sufficient amount of electrical current has been applied to stop the bleeding, the power source 104 may be deactivated to cease the application of current to the tissue within the chamber 112, the suction source 206 may be deactivated to stop application of the suction, and the distal portion 110 may be distally withdrawn from the treatment site 700.

In some methods, although possible, the application of the electrical current to the base portion 708 may not circumferentially scar the base portion 708 as in the other previously described methods, but rather just enough to stop the bleeding of the bleeding portion 705. As such, when the power source 104 and the suction source 206 are deactivated, the target tissue portion 704 may largely retract back to its original position prior to being withdrawn into the distal chamber 112, the main difference being that the portion 705 that was bleeding may no longer be bleeding. In other methods, the electrical current and suction may be applied long enough to stop the bleeding such that a scarring does occur, leaving the target tissue portion 704 in a raised position, as with the previous methods. Increasing the length that electrical current is applied for an extended period of time may increase the zone of coagulation. As a result, use of suction to perform hemostasis may allow for less accurate placement of the electrodes relative to the bleeding tissue compared to other devices that use only application of electrical current.

In addition, use of suction and electrical current to stop the bleeding of tissue may, at least in some situations, be preferred to other methods that do not use suction. In those other methods, a mechanical force (pressure) may be applied by the electrodes and/or support structure supporting the electrodes to the bleeding tissue, which may cause perforation of the tissue. When perforation occurs, a subsequent surgical procedure may need to be performed to repair the damaged tissue. Applying suction to create contact between the electrodes and the tissue by proximally withdrawing the tissue into the distal chamber, rather than applying mechanical pressure to create the contact, may provide less of a risk of perforation, and therefore more desirable.

However, in both the hemostasis method described with reference to Figs. 7A-7C and the methods of raising a target tissue portion as described with reference to Figs. 2A-2G and 5A-5E, the suction and application of electrical current to the tissue does not detach a target tissue portion from the underlying tissue. Instead, the suction and application of electrical current is used to perform some other action on the target tissue portion inside the distal chamber, e.g., create a ring of scarring at the base or stop bleeding.

In some example methods, if the electrosurgical device 102 is not able to stop all of the bleeding with a single withdrawal of tissue into the distal chamber 112, the distal portion 110 may be repositioned and the method may be repeated to stop further bleeding.

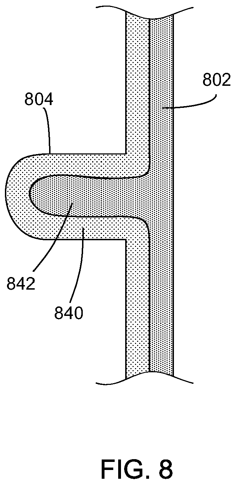

Fig. 8 shows an anatomical cross-sectional side view of underlying tissue 802 that includes a target tissue portion 804 in a raised position. The underlying tissue 802 and the target tissue portion 804 may be representative of any of the underlying tissues 202, 502, 702 and the target tissue portions 204, 504, 704 of Figs. 2A-2G, 5A-5E, 7A-7C, respectively. The underlying tissue 802 shown in Fig. 8 may include two layers of the gastrointestinal wall, namely the mucosa layer 840 and the submucosa layer 842 of the gastrointestinal wall, which may be the two closest tissue layers of the gastrointestinal wall closest to an inner lumen defined by and extending through the gastrointestinal wall. When suction is applied to the underlying tissue 802, at most, the mucosa layer 840 and the submucosa layers 842 may be part of the target tissue portion 804 and drawn to within a distal chamber of the electrosurgical medical device. That is, other tissue layers of the gastrointestinal wall, such as the muscularis externa or the serosa layers of the gastrointestinal wall (not shown in Fig. 8), may remain part of the underlying tissue 802 and not drawn to within the distal chamber. In some methods, the target tissue portion 804 may include only the mucosa layer 840, while in other methods, the target tissue portion 804 may include both the mucosa layer 840 and the submucosa layer 842, but not the other layers. Isolating and applying electrical current to only the mucosa layer 840 or only the mucosa and submucosa layers 840, 842 and not the other layers of the gastrointestinal wall may diminish the chances of causing perforation (e.g., delayed perforation) of the gastrointestinal wall due to the application of the electrical current.

The foregoing description of various embodiments of the invention has been presented for purposes of illustration and description. It is not intended to be exhaustive or to limit the invention to the precise embodiments disclosed. Numerous modifications or variations are possible in light of the above teachings. The embodiments discussed were chosen and described to provide the best illustration of the principles of the invention and its practical application to thereby enable one of ordinary skill in the art to utilize the invention in various embodiments and with various modifications as are suited to the particular use contemplated. All such modifications and variations are within the scope of the invention as determined by the appended claims when interpreted in accordance with the breadth to which they are fairly, legally, and equitably entitled.

## Claims

1. An electrosurgical system (300) comprising:
an elongate tubular member extending from a proximal portion to a distal portion, the tubular member comprising:
a body longitudinally extending from the proximal portion to the distal portion;
a distal chamber (312) disposed within the body at the distal portion; and
a snare lumen longitudinally extending in the body and in fluid communication with the distal chamber;
a deployable electrosurgical snare (210) comprising a distal loop portion (212), the distal loop portion movable between an undeployed position and a deployed position, wherein, in the undeployed position, the distal loop portion is at least partially disposed in the snare lumen extending in the body, and in the deployed position, the distal loop portion is disposed within the distal chamber circumferentially adjacent an inner surface of the body defining the distal chamber;
wherein the distal loop portion in the deployed position is disposed at a distal portion of the distal chamber for contact with a base of a target tissue portion of underlying tissue that is withdrawn into the distal chamber;
**characterized by**:
a return electrode (326) in the distal chamber that is at least partially circumferentially disposed on the inner surface,
wherein the return electrode is disposed at a distal portion of the distal chamber for contact with the base of said target tissue portion.

2. The electrosurgical system of claim 1, wherein the elongate tubular member further comprises a suction lumen longitudinally extending in the body, and wherein the distal chamber is in fluid communication with and disposed distal the suction lumen.

3. The electrosurgical system of claims 1 or 2, wherein the distal loop portion, in the deployed position, is disposed distal the return electrode.

4. The electrosurgical system of any of claims 1 to 3, wherein the elongate tubular member further comprises a lip that decreases a diameter of the distal chamber at a distal opening of the distal chamber to prevent the distal loop portion from slipping outside of the distal chamber.

5. The electrosurgical system of any of claims 1 to 4, wherein the return electrode comprises a ring-shaped structure.

6. The electrosurgical system of claim 5, wherein when the distal loop portion is in the deployed position, the distal loop portion and the return electrode are oriented in substantially parallel planes.

## Patentansprüche

1. Elektrochirurgisches System (300), umfassend:
ein längliches rohrförmiges Element, das sich von einem proximalen Abschnitt zu einem distalen Abschnitt erstreckt, wobei das rohrförmige Element umfasst:
einen Körper, der sich in Längsrichtung von dem proximalen Abschnitt zu dem distalen Abschnitt erstreckt;
eine distale Kammer (312), die innerhalb des Körpers an dem distalen Abschnitt angeordnet ist; und
ein Schlingenlumen, das sich in Längsrichtung in dem Körper und in Fluidkommunikation mit der distalen Kammer erstreckt;
eine ausbringbare elektrochirurgische Schlinge (210), die einen distalen Schlaufenabschnitt (212) umfasst, wobei der distale Schlaufenabschnitt zwischen einer nicht-ausgebrachten Position und einer ausgebrachten Position bewegbar ist, wobei der distale Schlaufenabschnitt in der nicht-ausgebrachten Position mindestens teilweise in dem Schlingenlumen angeordnet ist, das sich im Körper erstreckt, und der distale Schlaufenabschnitt in der ausgebrachten Position innerhalb der distalen Kammer im Umfang angrenzend an eine Innenfläche des Körpers angeordnet ist, der die distale Kammer definiert;
wobei der distale Schlaufenabschnitt in der ausgebrachten Position an einem distalen Abschnitt der distalen Kammer zum Kontakt mit einer Basis eines Zielgewebeabschnitts von darunter liegendem Gewebe angeordnet ist, das in die distale Kammer gezogen wird;
**gekennzeichnet durch**:
eine Gegenelektrode (326) in der distalen Kammer, die mindestens teilweise im Umfang auf der Innenfläche angeordnet ist,
wobei die Gegenelektrode an einem distalen Abschnitt der distalen Kammer zum Kontakt mit der Basis des Zielgewebeabschnitts angeordnet ist.

2. Elektrochirurgisches System nach Anspruch 1, wobei das längliche rohrförmige Element ferner ein Sauglumen umfasst, das sich in Längsrichtung im Körper erstreckt, und wobei die distale Kammer in Fluidkommunikation mit dem Sauglumen steht und distal desselben angeordnet ist.

3. Elektrochirurgisches System nach Anspruch 1 oder 2, wobei der distale Schlaufenabschnitt in der ausgebrachten Position distal der Gegenelektrode angeordnet ist.

4. Elektrochirurgisches System nach einem der Ansprüche 1 bis 3, wobei das längliche rohrförmige Element ferner eine Lippe umfasst, die einen Durchmesser der distalen Kammer an einer distalen Öffnung der distalen Kammer verringert, um zu verhindern, dass der distale Schlaufenabschnitt aus der distalen Kammer rutscht.

5. Elektrochirurgisches System nach einem der Ansprüche 1 bis 4, wobei die Gegenelektrode eine ringförmige Struktur umfasst.

6. Elektrochirurgisches System nach Anspruch 5, wobei der distale Schlaufenabschnitt und die Gegenelektrode in im Wesentlichen parallelen Ebenen orientiert sind, wenn der distale Schlaufenabschnitt in der ausgebrachten Position ist.

## Revendications

1. Système électrochirurgical (300) comprenant :
un élément tubulaire allongé s'étendant depuis une partie proximale vers une partie distale, l'élément tubulaire comprenant :
un corps s'étendant longitudinalement depuis la partie proximale vers la partie distale ;
une chambre distale (312) disposée dans le corps au niveau de la partie distale ; et
une lumière d'anse s'étendant longitudinalement dans le corps et en communication fluidique avec la chambre distale ;
une anse électrochirurgicale déployable (210) comprenant une partie boucle distale (212), la partie boucle distale étant mobile entre une position non déployée et une position déployée, dans laquelle, dans la position non déployée, la partie boucle distale est au moins partiellement disposée dans la lumière d'anse s'étendant dans le corps, et dans la position déployée, la partie boucle distale est disposée dans la chambre distale de façon adjacente circonférentiellement à une surface interne du corps délimitant la chambre distale ;
dans lequel la partie boucle distale dans la position déployée est disposée au niveau d'une partie distale de la chambre distale pour un contact avec une base d'une partie de tissu cible d'un tissu sous-jacent qui est tiré dans la chambre distale ;
**caractérisé par** :
une électrode de retour (326) dans la chambre distale qui est au moins partiellement disposée circonférentiellement sur la surface interne,
l'électrode de retour étant disposée au niveau d'une partie distale de la chambre distale pour un contact avec la base de ladite partie tissu cible.

2. Système électrochirurgical selon la revendication 1, dans lequel le élément tubulaire allongé comprend en outre une lumière d'aspiration s'étendant longitudinalement dans le corps, et dans lequel la chambre distale est en communication fluidique avec la lumière d'aspiration et disposée distalement à celle-ci.

3. Système électrochirurgical selon la revendication 1 ou 2, dans lequel la partie boucle distale, dans la position déployée, est disposée distalement à l'électrode de retour.

4. Système électrochirurgical selon l'une quelconque des revendications 1 à 3, dans lequel l'élément tubulaire allongé comprend en outre une lèvre qui diminue un diamètre de la chambre distale au niveau d'une ouverture distale de la chambre distale pour empêcher que la partie boucle distale glisse en dehors de la chambre distale.

5. Système électrochirurgical selon l'une quelconque des revendications 1 à 4, dans lequel l'électrode de retour comprend une structure de forme annulaire.

6. Système électrochirurgical selon la revendication 5, dans lequel lorsque la partie boucle distale est dans la position déployée, la partie boucle distale et l'électrode de retour sont orientées dans des plans sensiblement parallèles.
